# EUROPÄISCHE PATENTSCHRIFT

(11) **EP 0 464 359 B1**
(45) Veröffentlichungstag und Bekanntmachung des Hinweises auf die Patenterteilung: **19.04.1995**
(21) Anmeldenummer: 91108502.5
(22) Anmeldetag: 25.05.1991
(51) Int. Cl.: C07D 207/267

(54) **Verfahren zur Herstellung von N-substituierten Pyrrolidonen**
Process for the preparation of N-substituted pyrrolidones
Procédé pour la préparation de pyrrolidones N-substitués

(30) Priorität: 07.06.1990 DE 4018242
(43) Veröffentlichungstag der Anmeldung: 08.01.1992
(73) Patentinhaber: BASF Aktiengesellschaft, 67063 Ludwigshafen (DE)
(72) Erfinder: Koehler, Ulrich, Dr., W-6900 Heidelberg (DE); Siegel, Hardo, Dr., W-6720 Speyer (DE)

(56) Entgegenhaltungen:
- EP-A- 0 304 696
- US-A- 3 109 005

## Beschreibung

Die vorliegende Erfindung betrifft ein Verfahren zur Herstellung Von N-substituierten Pyrrolidonen durch Hydrierung von Gemischen aus Maleinsäureanhydrid, Maleinsäure und/oder Fumarsäure in Gegenwart von Ammoniak und mindestens sröchiometrischen Mengen eines Alkohols oder Aldehyds und eines Katalysators, der überwiegend Cobalt und mindestens eines der Elemente Mangan, Kupfer, Phosphor, Molybdän und/oder Natrium enthält.

Im folgenden Text werden die nachstehenden Abkürzungen benutzt:
MSA für Maleinsäureanhydrid, BSA für Bernsteinsäureanhydrid, MS für Maleinsäure, FS für Fumarsäure, BS für Bernsteinsäure und NMP für N-Methylpyrrolidon.

Aus der US-A-3,109,005 ist bekannt, Gemische aus MSA und Methylamin in Gegenwart von Raney-Nickel zu N-Methylpyrrolidon (NMP) zu hydrieren. Bei 270°C und 250 bar, Dioxan als Lösungsmittel und Reaktionszeiten von 10 Stunden erzielt man diskontinuierlich im Autoklaven NMP-Ausbeuten von 70%.

Aus der DE-A-22 00 600 ist weiterhin bekannt, MSA/Methylamingemische in Gegenwart von Palladium-Trägerkatalysatoren zu NMP zu hydrieren. Die höchsten NMP-Ausbeuten werden mit 44% bei 275°C und 120 bar in Wasser als Lösungsmittel erreicht. Bemerkenswert ist, daß unter fast gleichen Reaktionsbedingungen im System MSA/Ammoniak/Wasser 2-Pyrrolidon-Ausbeuten von 78% erzielbar sind. NMP ist demnach mit wesentlich niedrigerer Ausbeute herstellbar als 2-Pyrrolidon.

Aufgrund dieser mäßigen oder ungenügenden Ausbeuten sind diese Verfahren gegenüber dem herkömmlichen Verfahren zur NMP-Herstellung, das ist die Umsetzung von γ-Butyrolacton mit Methylamin, nicht konkurrenzfähig und haben keine technische Bedeutung erlangt.

N-subtituierte Pyrrolidone, insbesondere NMP, werden in großen Mengen als Lösungs- und Extraktionsmittel verwendet und produziert. MSA ist eine in großen Mengen verfügbare, preiswerte Grundchemikalie. Es bestand daher die Aufgabe, ein wirtschaftliches Verfahren zur Direktsynthese von N-substituierten Pyrrolidonen durch die katalytische Hydrierung von MSA-Amin-Mischungen zu entwickeln. Insbesondere sollte ein Katalysator zur Verfügung gestellt werden, der die Herstellung der genannten Pyrrolidone in guten Ausbeuten ermöglicht und sich durch eine hohe Katalysatorstandzeit auszeichnet.

Dementsprechend wurde ein Verfahren zur Herstellung von N-substituierten Pyrrolidonen durch die katalytische Hydrierung von Maleinsäureanhydrid, Maleinsäure und/oder Fumarsäure in Gegenwart von Ammoniak gefunden, das dadurch gekennzeichnet ist, daß man mindestens stöchiometrische Mengen eines primären Alkohols oder eines Aldehyds verwendet und einen Katalysator, der Cobalt und mindestens eines der Elemente Mangan, Kupfer, Phosphor, Molybdän und/oder Natrium enthält.

Die den erfindungsgemäßen Verfahren zugrundeliegende Hydrierung von MSA(I)-Ammoniak-Mischungen zu N-substituierten Pyrrolidonen (II) läßt sich formal durch die folgende Reaktionsgleichung beschreiben: 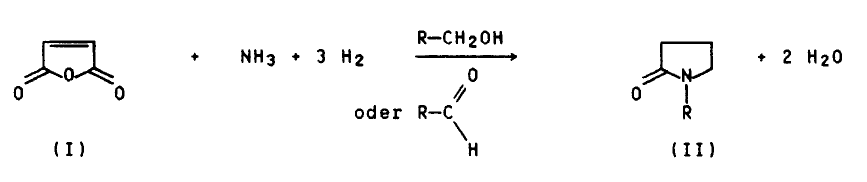
Der Verwendung von MSA als Einsatzstoff im erfindungsgemäßen Verfahren ist die Verwendung von MS und/oder FS äquivalent. Alle diese Einsatzstoffe können in fester, flüssiger oder gasförmiger Form in das erfindungsgemäße Verfahren eingebracht werden. Besonders bevorzugt ist die Verwendung von MSA. Vorteilhafterweise wird gasförmiges MSA, wie es in der Regel im technischen Maßstab bei der katalytischen Oxidation von Butan, Buten oder aromatischen Kohlenwasserstoffen gebildet wird, in einem Lösungsmittel absorbiert und die erhaltene Lösung ohne weitere Aufarbeitung der hydrierenden Umsetzung zugeführt.

Erfindungsgemäß werden zur katalytischen Hydrierung der MSA-Ammoniak-Mischungen Katalysatoren verwendet, die Cobalt und mindestens eines der Elemente Mangan, Kupfer, Phosphor, Molybdän und/oder Natrium enthalten. Bevorzugt werden Katalysatoren benutzt, welche neben Cobalt noch mindestens zwei der Elemente Mangan, Kupfer, Phosphor, Molybdän und/oder Natrium enthalten. Durch besonders vorteilhafte Eigenschaften zeichnen sich im erfindungsgemäßen Verfahren solche Katalysatoren aus, die neben Cobalt mindestens drei der Elemente Mangan, Kupfer, Phosphor, Molybdän und/oder Natrium enthalten. Solche Katalysatoren sowie ihre Herstellung sind in DE-A 23 21 101 und in der DE-A-39 04 083 beschrieben.

Vorteilhaft können im erfindungsgemäßen Verfahren beispielsweise solche Katalysatoren angewandt werden, deren katalytisch aktive Masse zu mindestens 40 Gew.-% aus Cobalt (ber. als Co) besteht und als weitere aktive Bestandteile bis zu 10 Gew.-%, vorzugsweise 3 bis 7 Gew.-% Mangan (ber. als Mn) , bis zu 20 Gew.-%, vorzugsweise 0,1 bis 5 Gew.-% Phosphorsäure und bis zu 1 Gew.-%, vorzugsweise 0,01 bis 0,5 Gew.-% Natrium (ber. als Na) enthält. Besonders bevorzugt werden solche der vorgenannten Katalysatoren verwendet, deren katalytisch aktive Masse als zusätzliche, katalytisch aktive Bestandteile bis zu 30 Gew.-%, vorzugsweise 12 bis 18 Gew.-% Kupfer (ber. als Cu) und bis zu 5 Gew.-%, vorzugsweise 1 bis 4 Gew.-% Molybdän (ber. als Mo) enthalten.

Die erfindungsgemäß verwendeten Katalysatoren können im erfindungsgemäßen Verfahren sowohl als Trägerkatalysatoren oder vorzugsweise in kompakter Form, d.h. ohne Träger eingesetzt werden. Die Art des Trägermaterials ist in der Regel nicht kritisch, es können übliche Trägermaterialien wie Siliciumdioxid, Aluminiumoxide, Titandioxide, Aktivkohle, Silikate oder Zeolithe verwendet werden. Erforderlichenfalls können zur Herstellung der Katalysatoren Bindemittel oder Formhilfsmittel mitverwendet werden.

Die Katalysatoren werden vorzugsweise vor ihrem Einsatz im erfindungsgemäßen Verfahren mit Wasserstoff aktiviert. Dabei werden die nach der Calcinierung im allgemeinen in Form ihrer Oxide vorliegenden aktiven Katalysatorbestandteile größtenteils reduziert und zwar in der Regel zu den entsprechenden Metallen. Weitere Einzelheiten zur Herstellung dieser Katalysatoren können DE-A 23 21 101 sowie der DE-A-39 04 083 entnommen werden.

Die Katalysatoren können im erfindungsgemäßen Verfahren in suspendierter Form angewandt werden, bevorzugt ist jedoch eine Festbettanordnung des Katalysators, über die die Einsatzstoffe in der Sumpf- oder vorzugsweise der Rieselfahrweise geleitet werden.

Die erfindungsgemäße Hydrierung kann in Gegenwart eines Lösungsmittels durchgeführt werden. Als Lösungsmittel kommen aliphatische und cyclische Ether z.B. Diethylether, Methyl-tert.-butylether, Dioxan, Tetrahydrofuran und/oder die Reaktionsprodukte, z.B. N-Methylpyrrolidon, besonders bevorzugt Wasser in Betracht.

Das Molverhältnis von MSA. MS oder FS und Ammoniak zu den Lösungsmitteln beträgt 1 : 0,001 bis 1 : 100, insbesondere 1 : 5 bis 1 : 50. Die Reaktion kann jedoch auch lösungsmittelfrei durchgeführt werden.

Besonders vorteilhaft ist es, wenn man das MSA, MS oder FS aus den bei der Oxidation von n-Butan oder Butenen entstehenden Gasgemischen mit Wasser absorbiert und derartige Lösungen ohne weitere Aufarbeitung für die Hydrierung verwendet.

Als Reaktionspartner werden primäre Alkohole wie C₁- bis C₁₂-Alkanole, bevorzugt C₁- bis C₄-Alkanole, z.B. Methanol, Ethanol, n-Butanol, Cyclohexanol, Phenol oder Benzylalkohol bzw. Aldehyde wie C₁- bis C₁₂-Aldehyde, z.B. Formaldehyd, Acetaldehyd oder Benzaldehyd verwendet.

Das Molverhältnis von MSA, MS oder FS zu Ammoniak beträgt im allgemeinen : 1 : 1 bis 1 : 25, vorzugsweise 1 : 1 bis 1 : 5, besonders bevorzugt 1 : 1 bis 1 : 3.

Das Molverhältnis von Ammoniak zu Alkohol oder Aldehyd beträgt 1 : 1 bis 1 : 50, vorzugsweise 1 : 1 bis 1 : 20.

Die Hydrierung wird im allgemeinen bei Temperaturen von 100 bis 350°C, insbesondere 150 bis 300°C, durchgeführt. Man arbeitet bei Drücken von 50 bis 350 bar, insbesondere 100 bis 300 bar.

Um die hohe Hydrierwärme ohne Schädigung des Katalysators abzuführen, kann es sinnvoll sein, die Hydrierung auf zwei verschiendenen Temperatur- und/oder Druckniveaus durchzuführen. Eine Aufarbeitung nach dem ersten Temperatur-/Druckniveau erfolgt nicht. So kann man z.B. zunächst bei Temperaturen von 100 bis 220°C und Drücken von 50 bis 200 bar arbeiten, um dann die Hydrierung bei Temperaturen von 220 bis 300°C und Drücken von 200 bis 350 bar zu vervollständigen.

Die Hydrierung wird diskontinuierlich, insbesondere aber kontinuierlich, in einem oder mehreren Reaktoren durchgeführt. Man arbeitet in Sumpf-, insbesondere aber Rieselfahrweise, wobei der Katalysator fest angeordnet ist. Es ist aber auch möglich, einen suspendierten Katalysator einzusetzen. Man kann sowohl Wasserstoff als auch einen Teil des Hydrieraustrags in die Hydrierstufe zurückleiten.

Als Reaktoren können z.B. Rohrreaktoren oder Rohrbündelreaktoren verwendet werden, wobei die Reaktionswärme durch Außenkühlung oder im Fall von Rohrreaktoren durch Innenkühlung abgeführt werden kann. Als Reaktormaterialien sind z.B. normale Edelstähle, z.B. RV-Stähle geeignet.

Die Hydrierausträge enthalten neben den gewünschten N-substituierten Pyrrolidonen gegebenenfalls unsubstituiertes Pyrrolidon sowie kleine Mengen an (N-substituierten) Bernsteinsäureimiden, Bernsteinsäurediamiden, Bernsteinsäuremonoamiden und (N-substituierten) Pyrrolidinen. Die Aufarbeitung kann durch Destillation und/oder Extraktion erfolgen. Zu (N-substituierten) Pyrrolidonen hydrierbare Zwischenprodukte wie z.B. die genannten (N-substituierten) Bernsteinsäureimide und -amide können abgetrennt und in die Hydrierstufe zurückgeführt werden.

### Beispiele

### Beispiel 1

### Kontinuierliche Hydrierung von Diammoniummaleinat/Methanol

Die Hydrierung wurde in einem Rohrreaktor (2 m Länge, 16 mm Durchmesser) durchgeführt, der über einen außenliegenden Mantel mit Öl auf die erforderlichen Temperaturen aufgeheizt werden konnte. Gasförmiger und flüssiger Zulauf durchströmten den Reaktor gemeinsam von oben nach unten. Den Reaktoraustrag kühlte man auf Raumtemperatur und trennte ihn in einen flüssigen und gasförmigen Strom. Der verwendete Katalysator hatte die Zusammensetzung 66,8 Gew.-% CoO (= 52,7 Gew.-% Co-Gehalt), 19,1 Gew.-% CuO (= 15,3 Gew.-% Cu-Gehalt), 7,1 Gew.-% Mn₂O₃ (= 5,1 Gew.-% Mn-Gehalt), 3,3 Gew.-% MoO₃ (= 2,2 Gew.-% Mo-Gehalt), 3,5 Gew.-% H₃PO₄ (= 1,1 Gew.-% P-Gehalt) und 0,15 Gew.-% Na₂O (= 0,1 Gew.-% Na-Gehalt). Der Katalysator wurde als 2,5 -4,0-mm-Splitt eingesetzt und vor Beginn der Hydrierung mit Wasserstoff aktiviert.

Bei einem Gesamtdruck von 200 bar und einer Reaktorinnentemperatur von 250°C wurden dem Reaktor stündlich die in Tabelle 1 angegebenen Mengen Maleinsäurediammoniumsalz als 45%ige Lösung in Wasser sowie Methanol zugeführt (Umlauf 9 l/h). Die Analyse des flüssigen Reaktoraustrages (Destillation, GC) ergab folgende Zusammensetzung (Tabelle 1, die Angaben in Mol-% beziehen sich jeweils auf das eingesetzte Diammoniummaleinat).

| Zulauf Maleinsäure diammoniumsalzlösung (ml/h) | Zulauf Methanol (ml/h) | Mol-% | | | | |
|---|---|---|---|---|---|---|
| | | N-Methyl Pyrrolidon | Succcinimid Methyl-Succinimid | Pyrrolidon | Pyrrolidin Methylpyrrolidin | Bernsteinsäureamide, -salze |
| 90 | 45 | 43,4 | 5,7 | 41,6 | 3,1 | 2,2 |
| 90 | 90 | 80,3 | 4,9 | 5,8 | 2,9 | 2,1 |
| 135 | 135 | 60,5 | 11,2 | 18,7 | 1,0 | 5,3 |

### Beispiel 2

### Diskontinuierliche Hydrierung von Diammoniummaleinat/Methanol

In einem 300 ml Mini-Autoklav wurden 75 ml einer 45%igen wäßrigen Diammoniummaleinatlösung und 75 ml Methanol mit 10 g des in Beispiel 1 beschriebenen pulverisierten und aktivierten Katalysators 42 h bei 230°C hydriert. Der Austrag enthielt 89,1 mol% N-Methylpyrrolidon, 5,0 mol-% Pyrrolidin und Methylpyrrolidin sowie 2,7 mol-% Succinimid und Methylsuccinimid.

### Beispiel 3

### Diskontinuierliche Hydrierung von Diammoniummaleinat/Formaldehyd

Es wurde wie in Beispiel 2 beschrieben verfahren, jedoch an Stelle von 75 ml Methanol 150 ml 35%ige Formaldehydlösung eingesetzt. Man erhielt 65,3 mol-% N-Methylpyrrolidon, 12,5 mol-% Pyrrolidon, 6,1 mol-% Pyrrolidin und Methylpyrrolidin und 15,1 mol-% Succinimid und N-Methylsuccinimid.

### Beispiel 4

### Kontinuierliche Hydrierung von Diammoniummaleinat/Butanol

Ein 2 m-Rohrreaktor mit 41 mm Durchmesser wurde mit dem Katalysator der Zusammensetzung von Beispiel 1 (Stränge der Länge 7 mm und vom Durchmesser 4 mm) gefüllt und bei 250 bar Wasserstoffdruck und einem Flüssigkeitsumlauf von 50 l/h vom Reaktoreingang stündlich mit 500 ml 45%iger wäßriger Diammoniummaleinatlösung und 500 ml n-Butanol beschückt.

Der Reaktoraustrag wurde wie in Beispiel 1 beschrieben, aufgearbeitet. Man erhielt 57,3 mol-% N-Butylpyrrolidon, 20,7 mol-% Pyrrolidon, 5,1 mol-% Pyrrolidin und Butylpyrrolidin, 7,2 mol-% Succinimid und Butylsuccinimid und 5,1 mol-% Bernsteinsäureamide und Salze.

## Patentansprüche

1. Verfahren zur Herstellung von N-substituierten Pyrrolidonen durch katalytische Hydrierung von Maleinsäureanhydrid, Maleinsäure und/oder Fumarsäure in Gegenwart von Ammoniak, dadurch gekennzeichnet, daß man mindestens stöchiometrische Mengen eines primären Alkohols oder eines Aldehyds verwendet und einen Katalysator, der Cobalt und mindestens eines der Elemente Mangan, Kupfer, Phosphor, Molybdän und/oder Natrium enthält.

2. Verfahren nach Anspruch 1, dadurch gekennzeichnet, daß man als primäre Alkohole C₁- bis C₁₂-Alkanole oder als Aldehyde solche mit C₁- bis C₁₂ verwendet.

3. Verfahren nach Anspruch 1, dadurch gekennzeichnet, daß die aktive Masse des Katalysators mindestens zu 40 Gew.-% aus Cobalt besteht.

4. Verfahren nach Anspruch 1, dadurch gekennzeichnet, daß man einen Katalysator verwendet, dessen aktive Masse mindestens zu 40 Gew.-% aus Cobalt besteht und als weitere aktive Bestandteile bis zu 10 Gew.-% Mangan, bis zu 20 Gew.-% Phosphorsäure und bis zu 1 Gew.-% Natrium enthält.

5. Verfahren nach Anspruch 1, dadurch gekennzeichnet, daß man einen Katalysator verwendet, dessen aktive Masse mindestens zu 40 Gew.-% aus Cobalt besteht und als weitere aktive Bestandteile bis zu 10 Gew.-% Mangan, bis zu 30 Gew.-% Kupfer, bis zu 5 Gew.-% Molybdän, bis zu 20 Gew.-% Phosphorsäure und bis zu 1 Gew.-% Natrium enthält.

6. Verfahren nach Anspruch 1, dadurch gekennzeichnet, daß man ein Molverhältnis von MSA, Maleinsäure und/oder Fumarsäure (einschließlich eventuell vorhandener Folgeprodukte) zu Ammoniak von 1 : 1 bis 1 : 5, vorzugsweise 1 : 1 bis 1 : 3 enthält.

7. Verfahren nach Anspruch 1, dadurch gekennzeichnet, daß man ein Molverhältnis von Ammoniak zu Alkohol oder Aldehyd von 1 : 1 bis 1 : 50, vorzugsweise von 1 : 1 bis 1 : 20 einhält.

8. Verfahren nach Anspruch 1, dadurch gekennzeichnet, daß man als Alkohol Methanol oder als Aldehyd Formaldehyd verwendet.

9. Verfahren nach Anspruch 1, dadurch gekennzeichnet, daß man die Hydrierung bei Temperaturen von 100 bis 350°C, insbesondere 150 bis 300°C und Drücken von 50 bis 350 bar, insbesondere 100 bis 300 bar durchführt.

10. Verfahren nach Anspruch 1, dadurch gekennzeichnet, daß man die Hydrierung auf zwei verschiedenen Temperatur- und Druckniveaus durchführt, wobei man zunächst bei Temperaturen von 100 bis 220°C und Drücken von 50 bis 200 bar arbeitet, anschließend die Hydrierung bei Temperaturen von 220 bis 300°C und Drücken von 200 bis 350 bar vervollständigt.

## Claims

1. A process for the preparation of N-substituted pyrrolidones by catalytic hydrogenation of maleic anhydride, maleic acid and/or fumaric acid in the presence of ammonia, which comprises using at least stoichiometric amounts of a primary alcohol or of an aldehyde and using a catalyst which comprises cobalt and at least one of the elements manganese, copper, phosphorus, molybdenum and/or sodium.

2. A process as claimed in claim 1, wherein the primary alcohols used are C₁- to C₁₂-alkanols or the aldehydes used are C₁- to C₁₂-aldehydes.

3. A process as claimed in claim 1, wherein the active material of the catalyst comprises at least 40 % by weight of cobalt.

4. A process as claimed in claim 1, wherein a catalyst is used whose active material comprises at least 40 % by weight of cobalt and contains, as further active constituents, up to 10 % by weight of manganese, up to 20 % by weight of phosphoric acid and up to 1 % by weight of sodium.

5. A process as claimed in claim 1, wherein a catalyst is used whose active material comprises at least 40 % by weight of cobalt and contains, as further active constituents, up to 10 % by weight of manganese, up to 30 5 by weight of copper, up to 5 % by weight of molybdenum, up to 20 % by weight of phosphoric acid and up to 1 % by weight of sodium.

6. A process as claimed in claim 1, wherein a molar MAA, maleic acid and/or fumaric acid (including any secondary products present) to ammonia ratio of from 1 : 1 to 1 : 5, preferably from 1 : 1 to 1 : 3, is used.

7. A process as claimed in claim 1, wherein a molar ammonia to alcohol or aldehyde ratio of from 1 : 1 to 1 : 50, preferably from 1 : 1 to 1 : 20, is used.

8. A process as claimed in claim 1, wherein the alcohol used is methanol or the aldehyde used is formaldehyde.

9. A process as claimed in claim 1, wherein the hydrogenation is carried out at from 100 to 350°C, in particular at from 150 to 300°C, and at from 50 to 350 bar, in particular at from 100 to 300 bar.

10. A process as claimed in claim 1, wherein the hydrogenation is carried out at two different temperature and pressure levels, first at from 100 to 220°C and at from 50 to 200 bar and subsequently completed at from 220 to 300°C and at from 200 to 350 bar.

## Revendications

1. Procédé de fabrication de pyrrolidones N-substituées par l'hydrogénation catalytique de l'anhydride maléique, de l'acide maléique et/ou de l'acide fumarique en présence d'ammoniac, caractérisé en ce que l'on utilise des proportions au moins stoechiométriques d'un aldéhyde ou d'un alcool primaire et un catalyseur qui contient du cobalt et au moins l'un des éléments manganèse, cuivre, phosphore, molybdène et/ou sodium.

2. Procédé suivant la revendication 1, caractérisé en ce que, à titre d'alcools primaires, on utilise des alcanols en C₁ à C₁₂, ou, à titre d'aldéhydes, ceux qui comportent de 1 à 12 atomes de carbone.

3. Procédé suivant la revendication 1, caractérisé en ce que la masse active du catalyseur se compose pour au moins 40% en poids de cobalt.

4. Procédé suivant la revendication 1, caractérisé en ce que l'on utilise un catalyseur dont la masse active se compose pour au moins 40% en poids de cobalt et qui contient, à titre d'autres constituants actifs, jusqu'à 10% en poids de manganèse, jusqu'à 20% en poids d'acide phosphorique et jusqu'à 1% en poids de sodium.

5. Procédé suivant la revendication 1, caractérisé en ce que l'on utilise un catalyseur dont la masse active se compose pour au moins jusqu'à 40% en poids de cobalt et contient, à titre d'autres constituants actifs, jusqu'à 10% en poids de manganèse, jusqu'à 30% en poids de cuivre, jusqu'à 5% en poids de molybdène, jusqu'à 20% en poids d'acide phosphorique et jusqu'à 1% en poids de sodium.

6. Procédé suivant la revendication 1, caractérisé en ce que l'on maintient un rapport molaire de l'anhydride maléique, de l'acide maléique et/ou de l'acide fumarique (y compris d'éventuels produits de décomposition présents) à l'ammoniac de 1:1 à 1:5, de préférence 1:1 à 1:3.

7. Procédé suivant la revendication 1, caractérisé en ce que l'on maintient un rapport molaire de l'ammoniac à l'alcool ou à l'aldéhyde de 1:1 à 1:50, de préférence de 1:1 à 1:20.

8. Procédé suivant la revendication 1, caractérisé en ce que l'on utilise le méthanol à titre d'alcool et le formaldéhyde à titre d'aldéhyde.

9. Procédé suivant la revendication 1, caractérisé en ce que l'on entreprend l'hydrogénation à des températures de 100 à 350°C, plus particulièrement 150 à 300°C et sous des pressions de 50 à 350 bars, plus particulièrement 100 à 300 bars.

10. Procédé suivant la revendication 1, caractérisé en ce que l'on entreprend l'hydrogénation à deux niveaux différents de température et de pression où l'on travaille d'abord à des températures de 100 à 220°C et sous des pressions de 50 à 200 bars, puis on achève l'hydrogénation à des températures de 220 à 300°C sous des pressions de 200 à 250 bars.
